# EUROPÄISCHE PATENTANMELDUNG

(11) **EP 1 990 419 A1**
(43) Veröffentlichungstag der Anmeldung: **12.11.2008**
(21) Anmeldenummer: 07009426.3
(22) Anmeldetag: 10.05.2007
(51) Int. Cl.: C12P 19/04, C08B 37/00, C12N 1/06

(54) **Verfahren zur Isolierung von Glucan**

(71) Anmelder: Tex-a-tec AG, 9630 Wattwil (CH)
(72) Erfinder: Meyer, Martin, 8800 Thalwil (CH); Marte, Oliver, 9630 Wattwil (CH); Dutler, Hans, 8053 Zürich (CH)
(74) Vertreter: Schaad, Balass, Menzl & Partner AG

(57) **Zusammenfassung**

Die vorliegende Erfindung betrifft ein Verfahren zur Isolierung von Glucan, Protein, Mannan und Lipid aus Hefe. Dabei wird die Hefe in aufeinanderfolgenden Schritte bei steigenden Temperaturen mit Ultraschall behandelt. Die Verwendung der isolierten Produkte sowie eine Vorrichtung zur Durchführung des Verfahrens werden ebenfalls beschrieben

## Beschreibung

Die vorliegende Erfindung betrifft ein Verfahren zur Isolierung von Glucan aus Hefe. Sie betrifft ebenfalls die Vorrichtung zur Durchführung des Verfahrens, das isolierte Glucan sowie dessen Verwendung.

Als Biopolymere werden von lebenden Organismen aus gleichartigen Grundbausteinen aufgebaute Makromoleküle bezeichnet, die in der Natur als Gerüst- und Festigungsmaterial, aber auch als Energie- und Informationsspeicher in vielfältiger Zusammensetzung auftreten. Bekannte Beispiele sind Cellulose und Stärke, sowie die DNS (Desoxyribonucleinsäure als genetischer Informationsspeicher).

Solche Biopolymere gewinnen in den verschiedensten Gebieten (z.B. in der Pharmazie) immer mehr an Bedeutung. Die hauptsächlichen Gründe hierfür liegen wohl in ihren physikalischen, chemischen und biologischen Eigenschaften, unter anderem in ihrer Verträglichkeit für die Umwelt und den Menschen.

Biopolymere können unter anderem aus Pflanzen beziehungsweise deren Bestandteilen gewonnen werden. Eine mögliche Quelle ist die Zellwand von Hefen (Saccharomyces cerevisiae). Dieser einzellige zu den eukaryontischen Organismen zählende Pilz besitzt eine Grösse von ca. 2-5µm. β-1,3-Glucan (nachfolgend Glucan) ist ein Hauptbestandteil der Zellwand der Hefe (ungefähr 40%). Die Zusammensetzung und Struktur der Hefe-Zellwand ist bereits seit längerer Zeit bekannt. Die Struktur und die Anteile der Komponenten variieren stark aufgrund der gewählten Kultivierungsbedingungen (R. Bonaly, et al., Biochim. Biophys. Acta 244, (1971) S. 484 - 494).

Die typischen quantitativen Anteile einzelner Komponenten der Hefe-Zellwand stellen sich in Prozent der Trockensubstanz der Hefezellwand wie folgt dar, 55% Glucan, 19% Mannan, 15% Protein, 7% Lipid sowie 4% Chitin und andere(F.J. DiCarlo et al., Science 127, (1958) S. 756 - 757); D. J. Manners et. al., Biochem. J. 135, (1973) S. 19 - 36).

M. Osumi beschreibt Ultrastruktur von Hefezellwänden und die Bildung dieser Strukturen (M. Osumi, Micron, 29, 1998, S. 207-233).

Edelmann et al. beschreibt die Ultrastruktur und die chemische Zusammensetzung von Rhacocarpus purpurescens und die dazu verwendeten Zellwand-Fraktionierungsverfahren (Edelmann et al., Planta, 1998, 206, S. 315-321).

In seiner natürlichen Form weist Glucan einen hohen Polymerisationsgrad sowie eine Quartärstruktur, die durch ihre Poren charakterisiert ist, auf. Ausserdem findet man in der Hauptketten des Glucans die Triple Helix als Sekundärstruktur. Die Hauptketten bestehen hauptsächlich aus langen β-1,3-Glucan-Ketten, wogegen sich in den Seitenketten hauptsächlich relative kurze β-1,6-Glucan-Ketten finden (J.S. Bacon, et al., Biochem J. 114, (1969) S. 557 - 567).

Die Nutzung von Hefe als Rohstoff zur Isolierung von Glucan bietet sich insofern an, als die Hefe im Brauprozess vorerst als Abfallprodukt anfällt.

Hefe, insbesondere Bäckerhefe, wird bereits seit längerer Zeit verarbeitet. Im Zentrum des Interesses steht dabei in der Regel die Gewinnung der intrazellulären Stoffe (Autolysat). Die dabei angewandten Verfahren haben alle zumindest die teilweise oder sogar die weitgehende Zerstörung der Zellwand und damit des Glucans zur Folge.

Die bisher bekannten Verfahren zur Aufschliessung/Desintegration von Hefe lassen sich in mechanische, chemische und biologische Verfahren einteilen.

Die mechanischen Verfahren verwenden Nassmahlung in Rührwerksmühlen oder Hochdruckhomogenisatoren im industriellen Massstab.

M. J. Janusz et al (J. Immunol., 137, (1986), S. 3270-3276) beschreibt ein Verfahren zur Gewinnung von wasserlöslichem Glucan. Dabei wird Ultraschall zur Zerstörung der Partikelstruktur der Glucan enthaltenden Zellwände eingesetzt.

B. Balasundaram et al (Biotechnology Bioeng., 75, (2001), S. 607 - 614) beschreibt unter anderem die Verwendung von Hochdruckhomogenisation und Ultraschallbehandlung als Desintegrationsmethoden für Bakterien und Hefe und deren Einfluss auf die Freisetzungs-Kinetik von Enzymen.

C. Ruiz et al (Yeast, 15, (10B), (1999), S. 1001 - 1008) und V. Cid et al (Microbiol., 144, (1998), S. 25 - 36) beschreiben beide die Verwendung von Ultraschall, um die Auswirkung von Mutationen in Hefen auf die Stabilität der Zellwände zu testen.

K. W. Hunter et al (Lett. appl. Micobiol. 35, (2002) S. 267-271) beschreibt die Gewinnung von Glucan mittels einer chemischen Methode und den Einfluss von Ultraschallbehandlung auf die vorgängig erhaltenen Glucanpartikel.

F. M. Jordan et al (Patent US 6.476.003 B1, Nov. 5, 2002) offenbart die Herstellung von Glucanpartikeln mit einer Grösse von weniger als einem Mikrometer. Das gewonnene Glucan wird mit Ultraschall behandelt, um die Partikelgrösse zu reduzieren.

F. M. Klis in Yeast 10, S. 851-869 (1994)erwähnt die chemische und enzymatische Gewinnung von Zellwandkomponenten aus Hefe.

M. Sauter et al (WO 02/12348) offenbart die Isolierung von Glucanpartikeln. Das Verfahren vermeidet mechanische Desintegrationsmethoden, weist jedoch lange Behandlungszeiten, hohe Temperaturen sowie hohe pH-Werte auf. Ausserdem gelangen auch organische Lösungsmittel und Enzyme (Proteasen) zum Einsatz.

Praktisch allen aus dem Stand der Technik bekannten Verfahren zur Isolierung von Glucan haftet der Nachteil an, dass sie die teilweise oder gar vollständige Zerstörung der Hefezellwände nach sich ziehen. Verfahren, welche die Zellwände nicht ganz zerstören, haben zumindest eine drastische Änderung oder Zerstörung der Tertiär- und Quartärstruktur zur Folge. Die Ursache hierfür liegt in der Anwendung von ungeeigneten physikalischen/mechanischen und teilweise für Biomaterialien extremen chemischen Bedingungen (pH-Wert in Kombination mit hohen Temperaturen (T ≥ 90°C)). Die Anwendung hoher Temperaturen in Kombination mit hohen pH-Werten resultiert unweigerlich in der teilweisen Zerstörung der Tertiär- und der Quartärstruktur. Eine weitere Ursache dieser Zerstörung sind die langen Behandlungszeiten und die dabei auftretende oxidative Schädigung der Polysaccharide.

In der Folge wird unabhängig von allen weiteren Isolationsschritten ein Glucan erhalten, das eine Reinheit von ungefähr 95-97% aufweist, das aber kaum Strukturerhaltung während der Isolierung zu bieten vermag.

Mannan ist, wie weiter oben bereits erwähnt, eine weitere Komponente, die aus der Hefezellwand gewonnen werden kann. Hinsichtlich der Mannan-Gewinnung weisen die bekannten Methoden die nachteilige Konsequenz auf, dass Tenside im Zellwand-Extrakt durch unkontrollierte Verseifung der Triglyceride entstehen. Da das Mannan in Wasser löslich ist, gelingt dessen Abtrennung von den ebenfalls im Zellwand-Extrakt vorhandenen Lipiden und Proteinen nur durch Isolationsmethoden wie Ultrafiltration, Chromatographie oder fraktionierte Fällung. Tenside, ob intrinsisch gebildet oder als Reagenz zugeführt, können kaum mehr entfernt werden, interferieren jedoch mit all diesen Isolierungsmethoden.

Wird der Extraktionsschritt zwar mit hohen Temperaturen, aber bei pH-Werten im Neutralbereich durchgeführt, werden die Lipide und Proteine wegen der nicht stattfindenden Verseifung und dadurch ausbleibenden Tensid-Wirkung nur mangelhaft extrahiert. Dies hat die sehr ungünstige Folge, dass die in der Zellwand verbleibenden Proteine und Lipide durch aufwändige Nachbehandlungen entfernt werden müssen, die Proteine beispielsweise mittels enzymatischer Hydrolyse und die Lipide mittels Extraktion mit organischen Lösungsmitteln. Dabei besteht die Gefahr, dass die zugesetzten hydrolytischen Enzyme auch den Protein-Teil des Mannans zu hydrolysieren vermögen und so zu einem geschädigten Mannan führen. Überdies bringen diese Nachbehandlungen zusätzliche Substanzen in den Zellwand-Extrakt, die dann ihrerseits wieder das Mannan verunreinigen können.

Einen zusätzlichen grossen Nachteil, den Nachbehandlungen verursachen, sind die höheren Verfahrenskosten. Eine Verwendung des so gewonnenen Glucans im technischchemischen Bereich scheitert so auch aus Kostengründen.

Der wohl gewichtigste Nachteil hinsichtlich der Schädigung des Glucans entsteht ganz am Anfang der bekannten und lange dauernden Verfahren der Glucan-Isolierung, es ist die Autolyse der Hefezellen. Während dieser Phase haben die zusammen mit anderen proteolytischen Enzymen freigesetzten Glucanasen genügend Zeit in die Zellwand einzudringen und das Glucan mehr oder weniger stark und unkontrolliert zu hydrolysieren. Dieser Nachteil ist allen bekannten Verfahren inhärent, da die unkontrollierte Lyse der Hefezellen der erste Prozesschritt in allen bisherigen Glucan-Isolierungsverfahren ist.

Eine weitere, äusserst störende und alle Anwendungen des Glucans beeinträchtigende Erscheinung ist die Bildung von Maillard-Produkten. Diese Produkte führen beim Glucan zu einer gelblich-braunen Färbung und einem unangenehmen Geruch. Maillard-Produkte lassen sich kaum mehr aus dem Endprodukt entfernen. Proteine, Peptide oder Aminosäuren in Gegenwart von Kohlehydraten bilden bei hohen Temperaturen die strukturelle Grundlage für die Maillard-Reaktion. Bei allen bisherigen Verfahren kommen diese Bedingungen vor und führen damit unweigerlich zu diesen unerwünschten Nebenprodukten.

Aufgabe der vorliegenden Erfindung ist es, ein Verfahren bereitzustellen, das eine schnelle und weitgehend zerstörungsfreie Isolierung von Glucan aus Hefe gewährleistet. Das Verfahren ermöglicht gleichzeitig die Isolierung weiterer Komponenten aus der Hefezelle bzw. Hefezellwand.

Die Aufgabe wird gelöst durch ein Verfahren gemäss Anspruch 1, beziehungsweise durch die Erzeugnisse der Ansprüche 13 bis 17 sowie eine Vorrichtung gemäss Anspruch 18. Weitere bevorzugte Ausführungsformen sind Gegenstand der abhängigen Ansprüche.

Durch das erfindungsgemässe Verfahren kann Glucan aus Hefe in weitgehend intakter, partikulärer Form isoliert werden. Weitere Bestandteile der Hefe beziehungsweise der Hefezellwand werden durch das erfindungsgemässe Verfahren ebenfalls erhalten. Dies sind Mannan, Lysat, Protein und Lipide. Das erfindungsgemässe Verfahren kann sehr rasch durchgeführt werden und benötigt insgesamt weniger als 24 Stunden einschliesslich der nach jedem Zyklus benötigten Rüstzeit. Die kurze Dauer der einzelnen Verfahrensschritte und die milden Bedingungen in Bezug auf pH-Wert und Temperatur der einzelnen Verfahrensschritte führt zu einer grösstmöglichen Schonung der durch das Verfahren erhaltenen Produkte. Das gewonnene Glucan erfüllt höchste Qualitätsansprüche hinsichtlich Reinheit und Abwesenheit von Nebenprodukten. Die äusserst störenden Maillard-Produkte werden weitgehend vermieden. Zudem ist das erfindungsgemässe Verfahren sehr kostengünstig.

Das erfindungsgemässe Verfahren zur Isolierung von Glucan aus Hefe umfasst folgende Schritte, die nachfolgend näher beschrieben werden.

In einem Schritt a) wird eine Hefesuspension bei einer ersten Temperatur T1 und einem ersten pH-Wert pH1 mit Ultraschall behandelt und ein erster Feststoffs A abgetrennt.

In diesem Schritt wird ein C-Lysates ("Controlled Lysis) im vorwiegend mesophilen Temperaturbereich (T < 60°C) gewonnen.

In einem Schritt b) wird der im vorangehenden Schritt gewonnene Feststoff A wieder resuspendiert und anschliessend bei einer zweiten Temperatur T2 und einem zweiten pH-Wert pH2 mit Ultraschall behandelt. Danach wird ein Feststoff B abgetrennt. In diesem Schritt werden die Zellproteine im thermophilen Temperaturbereich (30 ≤ T ≤ 80°C) und niederen Alkalikonzentrationen (pH-Werte von 9-11) extrahiert.

In einem Schritt c) findet die Extraktion von Mannan und Lipiden im extrem thermophilen Temperaturbereich, (70 ≤ T ≤ 100 °C) unter Zusatz von Alkali zur gesteuerten Teilverseifung und quantitativen Isolierung der Lipide statt. Dazu wird der im vorangehenden Schritt gewonnene Feststoff B resuspendiert und bei einer dritten Temperatur T3 und einem dritten pH-Wert pH3 mit Ultraschall behandelt. Anschliessend wird ein Feststoff C abgetrennt.

Die Ultraschalleinwirkung besitzt eine zentrale Bedeutung im erfindungsgemässen Verfahren. Durch die Anwendung von Ultraschall wird der Stofftransport aus dem Zellinneren durch die Zellwand, die eine Stärke von ca. 30 nm besitzt und zu einem hohen Anteil aus Glucan besteht, erheblich beschleunigt. Durch die damit erzielte schnelle Extraktion des Vacuoleninhalts und der darin enthaltenen Enzyme kann der ansonsten parallel ablaufende enzymatische Abbau des Glucans weitgehend verhindert werden. Sehr wesentlich ist die Berücksichtigung des physikalischen Wirkungsprinzips des Ultraschalls in diesem Verfahren, um die Quartärstruktur des Glucans nicht zu schädigen. Der technisch ausgewiesene Ultraschallbereich beträgt 20 bis 10'000 KHz, womit im Wasser Schallwellen von 7.5 bis 0.015 cm generiert werden. Bei entsprechend hohem Energieeintrag in eine flüssige Phase, der im niedrigen Frequenzbereich höher ist (z.B. 20 KHz), entsteht Kavitation verbunden mit hohen Drücken und Temperaturen in den kollabierenden Gasbläschen. Der durch Ultraschall mögliche Energieeintrag in ein wässriges Medium besitzt ein von der Mediumstemperatur abhängiges Maximum (maximale Kavitation in Wasser ca. 30-40 °C). Bei Überschreitung dieser Temperatur verringert sich der mögliche Energieeintrag, weshalb die Extraktionstemperaturen so niedrig wie möglich zu halten sind (Steven V. Ley, et al., Ultrasound in Synthesis, Springer Verlag Berlin Heidelberg New York London Paris Tokyo Hong Kong (1989), S. 1-17). Die Kavitation entsteht bevorzugt an der Grenzphase Flüssigkeit/Feststoff und somit an der Zellwand der Hefe. Dadurch erklärt sich die massive Erhöhung der Transportgeschwindigkeit der Zellinhaltsstoffe durch die Zellwand und die gleichzeitige Gefahr der Zellwandschädigung durch Kavitationserscheinungen.

Im Rahmen der durch das Anlagenequipment vorgegebenen Verfahrenstechnik wird die Hefesuspension in der Anlage zirkuliert, wobei die Beschallungszeit eines zirkulierenden Volumenelementes, abhängig von den Volumenverhältnissen zwischen dem Speichertank und den Ultraschallreaktoren, 1-80%, vorzugsweise 5-30% der individuell vorgegebenen Zeit eines Prozessschrittes beträgt. Ein Prozessschritt besteht aus einer bis acht Prozesszyklen, wobei innerhalb eines Prozesszyklus die Hefesuspension im Anlagensystem einmal umgewälzt wird. Nach Abschluss eines Prozesszyklus im abgesetzten Anlagenbetrieb bzw. eines jeweiligen Prozessschrittes im kontinuierlichen Anlagenbetrieb wird die Biopolymersuspension in eine kontinuierlich betriebene Zentrifuge geleitet, um das Eluat, in dem sich je nach Prozessschritt die verschiedenen Zellextrakte befinden, von der Feststoffsuspension abzutrennen. Die Feststoffsuspension gelangt vorzugsweise in einen zweiten Speichertank von dem aus der nächste Extraktionszyklus gestartet wird. Auf diese Weise wird abwechslungsweise Tank (A) und Tank (B) benutzt. Die nach den jeweiligen Extraktionszyklen erhaltenen Eluate werden in eigenen Speichertanks bis zu deren Weiterverarbeitung gelagert.

Der Speichertank oder bei Verwendung mehrerer Tanks sind diese beheiz- und kühlbar, mit Dosierequipment und mit der zur Prozessregelung notwendigen Sensorik ausgestattet. Es sind dies mindestens die pH-Wert- und Temperaturkontrolle sowie das zur automatischen Wasser- und Reagenziendosierung notwendige Equipment. Zur Zirkulation der Biopolymersuspension steht eine zentral installierte Zirkulationspumpe zur Verfügung, die optional mit einem Durchflussmessgerät gekoppelt ist.

Je nach Menge der zu behandelnden Hefesuspension wird die Anlage mit einem oder mehreren, vorzugsweise in Serie geschalteten Ultraschallreaktoren betrieben. Die Anzahl und die Volumina der Reaktoren sind variable Grössen, die sich am zirkulierenden Hefesuspensionsvolumen orientieren, da die Beschallungszeit eines zirkulierenden Volumenelementes 1 - 80 %, bevorzugt 5 - 30 % der Behandlungszeit eines Extraktionsschrittes beträgt. Die auf die Hefemasse bezogene Raumbelastung der Ultraschallreaktoren beträgt 0.02 - 0.2 kg/(1 min) vorteilhafterweise 0.06 - 0.12 kg/(1 min).

Die zur Beschallung der Hefesuspension eingetragene Ultraschallleistung beträgt 20 -200 W/l vorzugsweise 25 - 50 W/l. Als Schallgeber können sowohl Schallköpfe als auch Rohrschwinger (Sonotroden) verwendet werden, die je nach Extraktionsstufe mit wechselnder Frequenz betrieben werden. Der typische Frequenzbereich zur Erzeugung von Kavitation in wässrigen Medien liegt bei 1 KHz bis 10 MHz vorzugsweise 10 bis 100 KHz, im Speziellen bei 20 - 50 KHz.

Die Anlage wird, ausgenommen von speziellen Forderungen an die extrahierten Produkte, anstelle eines kontinuierlichen Betriebs, absatzweise betrieben, da hierdurch einerseits weniger Wasser und andererseits höhere Wertstoffkonzentrationen in den Extrakten erzielt werden.

Die zur Glucanisolierung gelangende Hefesuspension enthält 5-30%, vorzugsweise 12-25%, Hefe. Diese wird in einem ersten Schritt vom Bieranteil abgetrennt, gewaschen und als 5-25%-ige Suspension für die Glucanisolierung zur Verfügung gestellt.

Der Verfahrensschritt a) besteht in der weitgehenden schonenden Isolierung der cytosolischen Bestandteile und der Isolierung der Vakuolen-Inhaltstoffe (C-Lysat). Dieser Verfahrensschritt wird diskontinuierlich in einem bis sechs Zyklen durchgeführt, je nach gefordertem Reinheitsgrad des nach diesem Schritt vorliegenden Glucans. Die Extraktionstemperatur der C-Lysatgewinnung liegt bei vorzugsweise 25-60 °C, besonders bevorzugt bei 40-50 °C. Der pH-Wert zur C-Lysatextraktion beträgt vorzugsweise 3-8, besonders bevorzugt 4-6.

Die Beschallungskonditionen der C-Lysatextraktion sind:
Beschallungszeit: 3-25% vorzugsweise 5-8% der Beschallungsdauer eines Extraktionszyklus
Ultraschallleistung (bezogen auf das Volumen des Ultraschallreaktors): 5-50 W/1, vorzugsweise 10-25 W/1
Schallfrequenz: 20-100 KHz, vorzugsweise 20-40 KHz

Mit den aufgeführten Bedingungen wurde für eine weitgehende C-Lysatextraktion eine überraschend kurze Extraktionszeit von nur 3-5 Stunden, vorzugsweise von ca. 4 Stunden erzielt, die zu einem ca. 55%-igen Gewichtsverlust der Hefe führte. In der heute praktizierten Arbeitsweise dauert die Extraktion des Autolysates 12-24 Stunden. Während dieser Zeit werden durch die freigesetzten Enzyme das Glucan sowie wertvolle Proteine und Lipide unter Verlust der geforderten Produkteigenschaften teilweise abgebaut.

Im Anschluss an jeden Extraktionszyklus erfolgt ein Filtrations- oder ein Zentrifugationsprozess zur Abtrennung des C-Lysates von den suspendierten Hefezellwänden. Der Hefefeststoffanteil wird erneut mit Wasser und allfälligen Reagenzien versetzt, um für den folgenden Extraktionsprozess erneut eine ca. 20 Gewichtsprozentige Suspension herzustellen.

Ähnlich der C-Lysatextraktion werden im der Verfahrensschritt b) mindestens zwei vorteilhafterweise vier bis acht Extraktionszyklen bei variablen Temperaturen durchgeführt. Ein erster Zyklus, allenfalls die ersten vier Zyklen werden bei vorzugsweise 30-80°C, besonders bevorzugt bei 50-60°C, mit einer ca. 20%-igen Feststoffsuspension durchgeführt. Im Rahmen dieser Extraktionsstufen werden vorwiegend Proteine isoliert. Der Proteinextraktionsgrad sollte höher 98% liegen, um die Bildung von Maillard-Reaktionsprodukten zu minimieren. Der pH-Wert der Biopolymersuspension während der Proteinextraktion liegt vorzugsweise bei 7-12, besonders bevorzugt bei 8-9.

Zur Erzielung maximaler Weissgrade des Glucans werden der Suspension im letzten Extraktionszyklus dieses Verfahrensschrittes, nachdem der Hauptanteil des Proteins extrahiert ist, optional 1-10 g/l einer nucleophilen Verbindung, z.B. Merkapto- und/oder aminofunktionelle Verbindungen vorzugsweise jedoch 2-4 g/l Natriumhydrogensulfit zugesetzt. Hierdurch werden die von den Glycoproteinen stammenden und an den Maillard-Reaktionen beteiligten Aldehydfunktionen (R-CHO; R= Glycoproteine und andere in Wasser lösliche Polysaccharide) blockiert, womit einerseits die Bildung von Maillard-Reaktionsprodukten minimiert und die Löslichkeit der Glycoproteine und Polysaccharide und damit deren Extraktionsgrad erhöht wird (siehe Reaktionsgleichung 1).

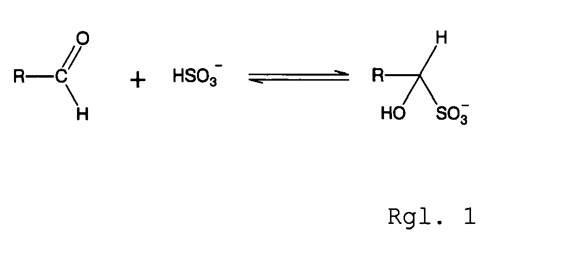

Eine andere optionale Möglichkeit zur Gewinnung eines sehr reinen Glucans ist der Zusatz von Natrium-Dodecylsulfat (SDS). Durch die Transacylierung werden negative Ladungen in die Proteinstruktur eingeführt, die die Löslichkeit der Proteine verbessern und vor allem eine Abtrennung in einem elektrischen Feld erlauben.

Nach diesem, auf eine oder andere Art optional durchgeführten Extraktionszyklus erfolgt ein bei pH 8-9 durchgeführter Spülzyklus, um die überschüssig eingesetzten Reagenzien zu eliminieren.

Die Beschallungsbedingungen der Proteinextraktion sind:
Beschallungszeit: 5-30%, vorzugsweise 8-15%, der Beschallungsdauer eines Extraktionszyklus
Ultraschallleistung (bezogen auf das Volumen des Ultraschallreaktors): 5-100 W/l, vorzugsweise 10-25 W/l.
Schallfrequenz: 20-100 KHz, vorzugsweise 20 - 40 KHz

Überraschenderweise wurde auch hier bereits nach einer zwei bis vierstündigen, vorzugsweise einer ca. dreistündigen, bei nur 60°C durchgeführten Extraktion eine nahezu vollständige Proteineliminierung (≥ 98%) festgestellt. In der heutigen Verfahrensweise dauert diese Prozessschritt 4-6 Stunden, bei Temperaturen von 80-120°C, verbunden mit der Bildung erheblicher Anteile von Maillard-Reaktionsprodukten, die zur Geruchsbildung und Bräunung des Glucans führen.

Anschliessend an die Proteinextraktion erfolgt der Verfahrensschritt c), die Lipid- und Mannanextraktion, verbunden mit der "Glucanaktivierung", in mindestens einem, vorteilhafterweise in zwei bis sechs Zyklen. Die Glucanaktivierung besteht in der funktionell wichtigen Hydratisierung und Quellung des Glucans, die zur Ausbildung der Porenstruktur führt.

Der Feststoffgehalt der mit Wasser eingestellten Suspension beträgt ca. 20 %, wobei nun nochmals 40 Gewichtsprozente des Feststoffanteiles zu extrahieren sind, um das reine Glucan zu erhalten. Die Temperatur während der Lipidextraktion beträgt vorzugsweise 70-100°C, besonders bevorzugt 80-90°C. Durch die Realisierung einer möglichst niederen Extraktionstemperatur wird die Bildung von Maillard-Reaktionsprodukten durch eventuell noch vorhandenes Restprotein minimiert. Der pH-Wert wird mit Natronlauge vorzugsweise auf pH 10-12, besonders bevorzugt auf 10.5-11.5, geregelt, wobei die Prozesszeiten möglichst kurz gewählt werden.

Überraschend wurde festgestellt, dass die unter den aufgeführten Reaktionsbedingungen (Temperatur, pH-Wert, Anzahl Extraktionszyklen), durch Verseifung der Triglyceride entstehenden Fettsäuresalze eine hervorragende Solvatisier- und Emulgierwirkung besitzen. Der Anteil des verseiften Lipidanteils beträgt 5-25% vorzugsweise 10-15 % bezogen auf den unverseiften Anteil. Der HLB-Wert (Hydrophilic-Lipophilic-Balance) der Fettsäuren liegt im Bereich von 14-18 und überdeckt in idealer Weise den HLB-Bereich der notwendig ist, um die Fette im Wasser zu solvatisieren und emulgieren.

Der Solvatisierungsprozess findet oberhalb der CMC (critical micelle concentration) der freigesetzten Fettsäuren und somit vorwiegend in den ersten Lipidextraktionszyklen statt (H. Sonntag, Lehrbuch der Kolloidwissenschaft, VEB Deutscher Verlag der Wissenschaften, Berlin (1977), S. 253-270). Die CMC der entstehenden Fettsäuren (C₁₆-C₁₈) liegt unter 10⁻³ mol/l, die im Bereich von 80°-100°C (Krafft-Punkt) überschritten wird. Mit abnehmender Lipidkonzentration in der Extraktionslösung werden die Fette vorwiegend emulgiert, wobei der Emulgierprozess durch die Beschallung erheblich begünstigt wird. Die angegebene Arbeitsweise bietet grosse Vorteile, da zum einen das Verteilungsgleichgewicht zu Gunsten des solubilisierten Lipidanteils verschoben wird und andererseits die Natriumsalze der gebildeten Fettsäuren nicht oder nur zu geringem Masse am Glucan sorbieren sowie keine System fremden Substanzen zur Emulgierung bzw. Solubilisierung eingesetzt werden müssen. Eine optionale Möglichkeit einen hohen Lipidextraktionsgrad zu erzielen ist der Einsatz von der Extraktionstemperatur angepassten Co-Tensiden, z.B. höher siedenden Alkoholen mit einer C-Kette von C₄-C₁₀ oder niedermolekularen Polypropylenderivaten (z.B. Pluriol P Typen der Firma BASF). Der Zusatz von Cotensiden erfolgt allenfalls im letzten Extraktionszyklus dieses Verfahresschrittes.

Die Beschallungsbedingungen der Mannan- und LipidExtraktion sind:
Beschallungszeit: 10-40% vorzugsweise 12-25% der Beschallungsdauer eines Extraktionszyklus
Ultraschallleistung (bezogen auf das Volumen des Ultraschallreaktors): 5-50 W/l, vorzugsweise 10-25 W/l
Schallfrequenz: 20-100 KHz, vorzugsweise 20-40 KHz

Die für den Verfahrensschritt c) unter Einsatz von Ultraschall benötigte Zeit zur vollständigen Mannan und Lipidisolierung beträgt nur drei bis vier Stunden bei 70-100°C im Vergleich mit den heute praktizierten Verfahren, die 4-6 Stunden dauern und bei 100-120°C durchgeführt werden.

Die weitere Aufarbeitung der Glucansuspension (z.B. Trocknung, chemische Modifikation etc. hängt vom späteren Verwendungszweck des Glucans ab.

In einer bevorzugten Ausführungsform wird die zur Glucangewinnung verwendete Hefe aus einem flüssigen Kulturmedium abgetrennt. Dies ist zum Beispiel das im Bierbrauprozess produzierte Bier oder die Bäckerhefe. Denkbar sind aber auch andere zur Kultivierung von Hefe geeignete Medien.

In einer weiteren bevorzugten Ausführungsform wird die Hefe mit Dampf, vorzugsweise Sattdampf, behandelt. Dies bewirkt die Desinfektion/Denaturierung der Hefe. Durch die weitgehende Enzymdenaturierung wird eine Qualitätsverbesserung des C-Lysates beziehungsweise Glucans erzielt. Dieser Verfahrensschritt, der bei höchsten Qualitätsansprüchen sowohl an das Glucan als auch an das C-Lysat durchgeführt wird, besteht in einer 2-15 Minuten, vorzugsweise 4-10 Minuten dauernden Dampfbehandlung der gewaschenen Hefe. Die Behandlung wird bei 100-110°C, vorteilhafterweise unter Sattdampfbedingungen (101-105°C, 1bar) durchgeführt. Diese bewirkt einerseits die Desinfektion der Hefe und der nachfolgend isolierten Hefeprodukte und andererseits die Deaktivierung/Denaturierung der Enzyme, womit die Gefahr des nachfolgenden Protein-, Lipid- und Polysaccharidabbaus weitgehend minimiert wird. Die aufgeführten Bedingungen der Dampfbehandlung führen vornehmlich zu einer Denaturierung der cytosolischen Proteine im Unterschied zu den in der Zellmembran fixierten Proteinen. Im Rahmen dieser selektiven Proteindenaturierung verlieren die Enzyme ihre Aktivität, während die Löslichkeit der Proteine unbeeinträchtigt bleibt.

In einer bevorzugten Ausführungsform wird der abgetrennte Feststoff C, das heisst, das isolierte Glucan, in wenigstens einem Zyklus gewaschen. Das Waschen erfolgt nach Abschluss der Protein- und Mannanextraktion. Besonders bevorzugt sind drei Waschzyklen des isolierten Glucans, wobei einer der Waschzyklen, vorzugsweise der zweite, ein mit Säure (z.B. Essig-, Salzsäure, etc.) geführter Neutralisationsprozess ist. Dabei wird ein annähernd neutraler pH-Wert eingestellt.

In einer bevorzugten Ausführungsform beträgt die erste Temperatur T1 25-60°C, besonders bevorzugt 40-50°C, und der erste pH-Wert 3-8, besonders bevorzugt 4-6.

In einer weiteren bevorzugten Ausführungsform beträgt die zweite Temperatur T2 30-80°C, besonders bevorzugt 50-60°C, und der zweite pH-Wert 7-12, besonders bevorzugt 8-9.

In einer bevorzugten Ausführungsform beträgt die dritte Temperatur T3 70-100°C, besonders bevorzugt 80-90°C, und der dritte pH-Wert 10-12, besonders bevorzugt 10.5-11.5.

In einer bevorzugten Ausführungsform werden in den Verfahrensschritten a), b) und c) steigende pH-Werte gewählt. Das heisst der zweite pH-Wert pH2 im Verfahrensschritt b) ist höher als der erster pH-Wert pH1 im Verfahrensschritt a) und der dritte pH-Wert pH3 im Verfahrensschritt c) ist höher ist als der zweite pH-Wert pH2. In dieser bevorzugten Ausführungsform gelten somit die beiden Bedingungen T1 < T2 < T3 und pH1 < pH2 < pH3.

Nach einer bevorzugten Ausführungsform wird als Hefe Brau- oder Bäckerhefe eingesetzt. Diese Hefe eignet sich besonders deshalb, weil sie im Brauprozess in hoher Qualität (Lebensmittelqualität) eingesetzt wird und in grösserer Menge anfällt.

In einer bevorzugten Ausführungsform wird das erfindungsgemässe Verfahren unter Schutzgas durchgeführt. Das Schutzgas minimiert die oxidative Schädigung der Hefe bzw. des Glucans und der übrigen Verfahrensprodukte. Als Schutzgase können Stickstoff und/oder Argon eingesetzt werden, wobei Stickstoff bevorzugt ist. Wird das Verfahren unter Schutzgas durchgeführt, sind der/die Speichertank(s) und die Ultraschallreaktoren bzw. die Vorrichtung zur Durchführung des Verfahrens geschlossen (in den Figuren nicht gezeigt).

Das isolierte partikuläre Glucan, welches nach dem erfindungsgemässen Verfahren hergestellt wird, ist im Schritt c) als Feststoff C abtrennbar.

Das C-Lysat, hergestellt nach dem erfindungsgemässen Verfahren, ist im Schritt a) vom Feststoff A abtrennbar.

Das Protein (Oligomere und Polymere), hergestellt nach dem erfindungsgemässen Verfahren ist im Schritt b) vom Feststoff B abtrennbar.

Das Mannan und die Lipide, welche nach dem erfindungsgemässen Verfahren hergestellt werden, ist im Schritt c) vom Feststoff C abtrennbar.

Das partikuläre Glucan, hergestellt nach dem erfindungsgemässen Verfahren, kann als Microcontainer verwendet werden. Es eignet sich hierzu ganz besonders aufgrund seiner intakten partikulären Struktur.

Eine Vorrichtung zur Durchführung des erfindungsgemässen Verfahrens umfasst einen Speichertank mit einem Rührwerk, eine Pumpe zur Beförderung einer Suspension und einen Ultraschallreaktor mit einem ultraschall-erzeugenden Mittel. Im Weiteren muss die Vorrichtung ein Mittel zur Einstellung der Temperatur, ein Mittel zur Einstellung des pH-Werts sowie ein Abtrennmittel zur Abtrennung eines Feststoffs umfassen. Eine Verbindungsleitung verbindet den Speichertank mit dem Ultraschallreaktor und eine Abfuhrleitung verbindet den Ultraschallreaktor mit dem Abtrennmittel.

Eine bevorzugte Ausführungsform der Vorrichtung umfasst mindestens zwei Ultraschallreaktoren. Besonders bevorzugt sind vier solcher Reaktoren. Die Ultraschallreaktoren sind dabei in Serie angeordnet und über Verbindungsleitungen miteinander verbunden. Dies erlaubt eine besonders effiziente Behandlung der Hefezell- bzw. Hefepartikelsuspension mit Ultraschall.

Eine weitere Ausführungsform der Vorrichtung zur Durchführung des erfindungsgemässen Verfahrens arbeitet kontinuierlich bezogen auf die innerhalb eines Prozessschrittes durchzuführenden Zyklen.

In dieser Ausführungsform umfasst die Vorrichtung einen Speichertank mit einem Rührwerk, der über Verbindungsleitungen mit in Serie angeordneten Ultraschallreaktoren mit einem ultraschall-erzeugenden Mittel verbunden. Nach den Ultraschallreaktoren ist ein Abtrennmittel zur Abtrennung eines Feststoffs angeordnet. Dieses Abtrennmittel ist über eine Abfuhrleitung und eine Rückführleitung mit den Ultraschallreaktoren. Im Weiteren muss die Vorrichtung ein Mittel zur Einstellung der Temperatur, ein Mittel zur Einstellung des pH-Werts und eine Pumpe zur Beförderung einer Suspension umfassen.

Weitere Merkmale, Einzelheiten und Vorzüge der Erfindung ergeben sich aus den nachfolgend beschriebenen Zeichnungen.

Es zeigen rein schematisch:
Fig. 1: Eine Ausführungsform einer Vorrichtung zur Durchführung des erfindungsgemässen Verfahrens;
Fig. 2: Eine bevorzugte Ausführungsform der Vorrichtung zur diskontinuerlichen oder absatzweisen Durchführung des erfindungsgemässen Verfahrens;
Fig. 3: Den Fluss der Suspension in einer kontinuierlich arbeitenden Vorrichtung zur Durchführung des erfindungsgemässen Verfahrens;
Fig. 4: Eine bevorzugte Ausführungsform der Vorrichtung zur kontinuierlichen Durchführung des erfindungsgemässen Verfahrens.

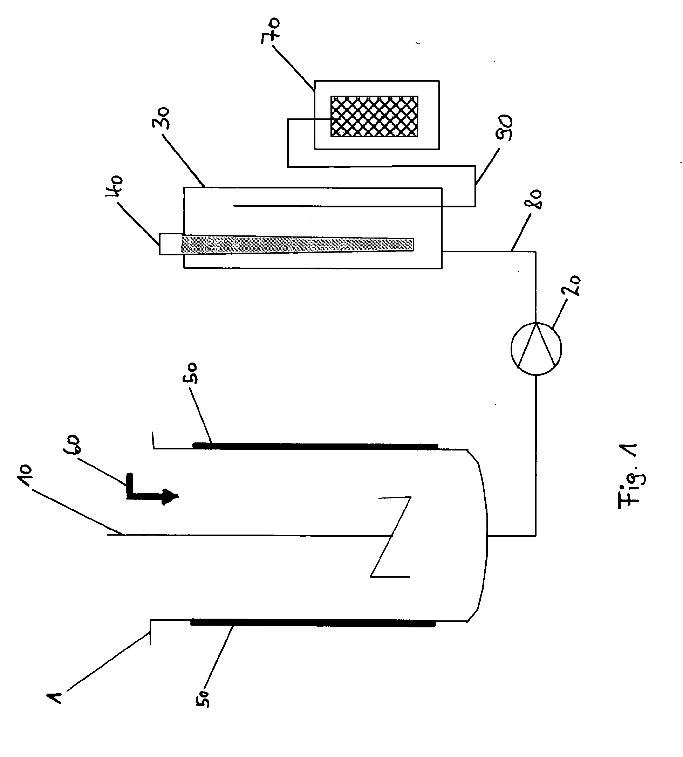

Figur 1 zeigt eine Vorrichtung zur Durchführung des erfindungsgemässen Verfahrens zur Isolierung von Glucan. Die Vorrichtung umfasst einen Speichertank 1 und ein darin zentral angeordnetes Rührwerk 10. Letzteres verhindert das unerwünschte Sedimentieren der Hefezell- bzw. Hefepartikelsuspension und damit möglicherweise einhergehendes Verstopfen von Leitungen. Eine Pumpe 20 befördert die im Speichertank befindliche Suspension (in der Figur nicht gezeigt) über eine Verbindungsleitung 80 in den Ultraschallreaktor 30, der ein ultraschallerzeugendes Mittel 40 umfasst. Das ultraschall-erzeugende Mittel 40 kann z.B. eine Sonotrode (Rohrschwinger) sein. Eingesetzt werden können jedoch auch andere ultraschall-erzeugende Mittel. Bei der Anordnung der Sonotroden ist zu beachten, dass Interferenzerscheinungen, die zu einer Verminderung oder gar Auslöschung des Ultraschalls führen können, möglichst vermieden werden. Dies lässt sich dadurch erreichen, dass die Sonotroden asymmetrisch, z.B. ausserhalb der Symmetrieachse im Falle eines kreiszylinderförmigen Ultraschallreaktors 30, angeordnet werden. Vom Ultraschallreaktor 30 führt eine Abfuhrleitung 90 zum Abtrennmittel 70. Als Abtrennmittel 70 können beispielsweise Filter oder auch Zentrifugen eingesetzt werden.

Der Speichertank 1 ist in der gezeigten Ausführungsform mit einem temperatur-einstellenden Mittel 50 ausgestattet. Dies ermöglicht es, die im Speichertank 1 befindliche Suspension zu erwärmen oder zu kühlen und nach Erreichen der gewünschten Temperatur, diese im Wesentlichen konstant zu halten. Ein solches temperatur-einstellendes Mittel kann z.B. ein an der Wand des Speichertanks angeordneter Hohlmantel sein, durch den eine Kühl- bzw. Heizflüssigkeit zirkuliert. Ein pH-einstellendes Mittel 60, das in der gezeigten Ausführungsform ebenfalls am Speichertank 1 angeordnet ist, dient der Regulierung des pH-Wertes der im Speichertank 1 befindlichen Supension. Der pH-Wert der Supension kann beispielsweise durch Zufuhr von Säure oder Lauge auf den gewünschten Wert eingestellt und nach Erreichen dieses Wertes im Wesentlichen konstant gehalten werden. Als Säure kann Salzsäure und als Lauge Natronlauge verwendet werden. Es sind auch organische Säuren und Basen denkbar, diese bilden jedoch oft Puffersysteme, die mit nachfolgenden Schritten interferieren können.

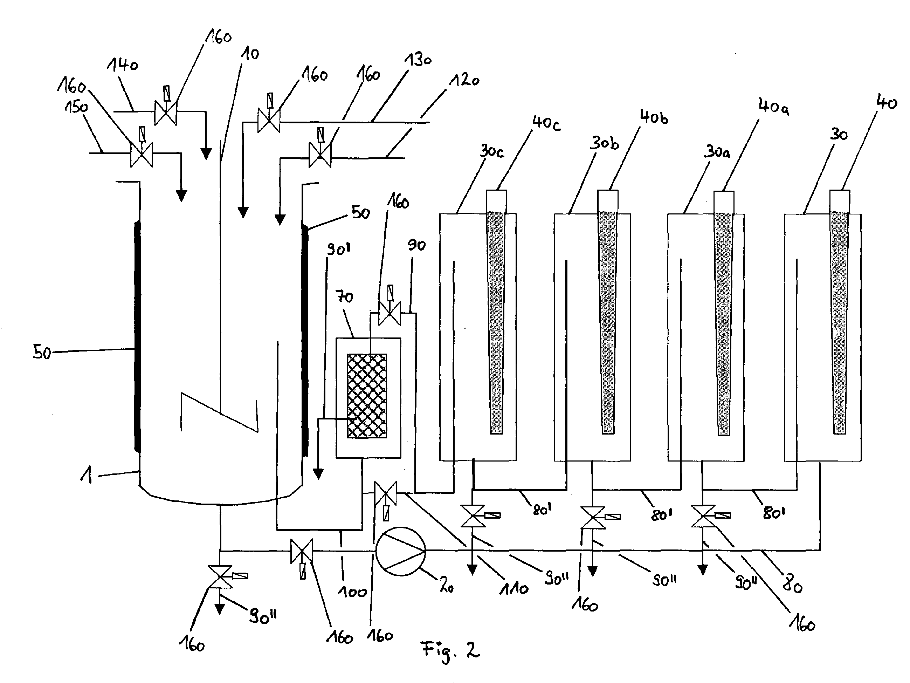

Figur 2 zeigt eine bevorzugte Ausführungsform der Vorrichtung zur Durchführung des erfindungsgemässen Verfahrens zur Isolierung von Glucan. Diese Ausführungsform umfasst wie die in der vorangehenden Figur 1 gezeigte Ausführungsform den Speichertank 1 mit dem zentral angeordneten Rührwerk 10.

Am Speichertank 1 sind zudem Zufuhrleitungen 120, 130, 140 und 150 angeordnet. Diese Zufuhrleitungen gestatten die Zugabe von Reagenzien, z.B. von Wasser, Dampf, Säure und/oder Lauge, zur Hefezell- bzw. Hefepartikelsuspension.

Das an der Wand des Speichertanks 1 angeordnete temperatur-einstellende Mittel 50 ermöglicht die Erwärmung oder Kühlung der im Speichertank 1 befindlichen Suspension (in der Figur nicht gezeigt'). Die Pumpe 20 befördert die Suspension über eine Verbindungsleitung 80 zum Ultraschallreaktor 30 mit dem ultraschall-erzeugenden Mittel 40. Der Ultraschallreaktor 30 ist mit einem weiteren in Serie angeordneten Ultraschallreaktor 30a über Verbindungsleitungen 80' verbunden. Der Ultraschallreaktor 30a ist seinerseits mit dem Ultraschallreaktor 30b über eine weitere Verbindungsleitung 80' verbunden. Der Ultraschallreaktor 30b ist schliesslich über eine weitere Verbindungsleitung 80' mit dem Ultraschallreaktor 30c verbunden. Die in Serie angeordneten Ultraschallreaktoren 30, 30a, 30b und 30c umfassen je das zugehörige ultraschall-erzeugende Mittel 40, 40a, 40b und 40c. Die serielle Anordnung der Ultraschallreaktoren erlaubt eine effiziente Behandlung der Hefezell- bzw. Hefepartikelsuspension. Der Ultraschallreaktor 30c ist über die Abfuhrleitung 90 mit dem Abtrennmittel 70 verbunden. Vom Abtrennmittel 70 führt eine weitere Abfuhrleitung 90' weg. Zudem ist das Abtrennmittel 70 über eine Rückfuhrleitung 100 mit dem Speichertank 1 verbunden. So besteht die Möglichkeit, die Hefezell- bzw. Hefepartikelsuspension vom Speichertank 1 über die in Serie angeordneten Ultraschallreaktoren 30, 30a, 30b und 30c wieder zurück in den Speichertank 1 zu führen. Die Abfuhrleitung 90 ist durch eine Umgehungsleitung 110 direkt mit der Rückfuhrleitung 100 verbunden. Damit ist eine direkte Verbindung zwischen dem Ultraschallreaktor 30c und dem Speichertank 1 unter Umgehung des Abtrennmittels 70 möglich.

Von den Verbindungsleitungen 80 und 80' der Ultraschallreaktoren 30, bzw. 30a, 30b und 30c führen zudem Abfuhrleitungen 90" weg.

Zur Steuerung des Flusses der Hefezell- bzw. der Hefepartikelsuspension sind die Verbindungsleitung 80, die Zufuhrleitungen 120, 130, 140 und 150, die Abfuhrleitungen 90, 90' und 90" sowie die Umgehungsleitung 110 mit Ventilen 160, z.B. elektrogesteuerten Ventilen ausgestattet.

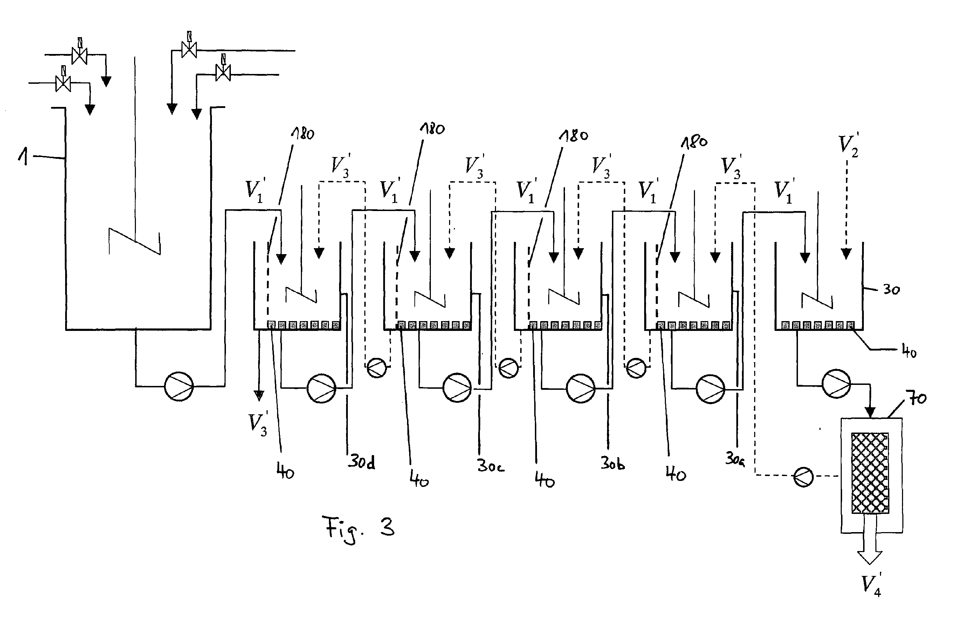

Figur 3 zeigt den Fluss der Suspension (Hefezell- bzw. Hefepartikelsuspension) in einer kontinuierlich arbeitenden Vorrichtung zur Durchführung des erfindungsgemässen Verfahrens.

Der Hefesuspensionstrom (Hefezell- bzw. Hefepartikelsuspension) mit der Flussrate V1' wird in der gezeigten Vorrichtung durch die in Serie angeordneten Ultraschallreaktoren 30, 30a, 30b, 30c und 30d geführt.

Die Hefesuspension befindet sich im Speichertank 1, der beheiz- und kühlbar ist. Sämtliche Reagenzienzugaben erfolgen sowohl in den Speichertank 1 als auch in den Extraktionslösungsstrom mit der Flussrate V₂'. Die Extraktion der zellulären Inhaltsstoffe erfolgt nach dem Gegenstromprinzip d.h. die Flussrichtung des Extraktionslösungstroms mit der Flussrate V₂', die nach dem Abtrennmittel 70 (Separator) zur Flussrate V₃' wird, ist in entgegengesetzter Richtung zum Hefesuspensionsstrom mit der Flussrate V₁'. Die Flussrate V₁' des Hefesuspensionstroms ist so einzustellen, dass die mittlere Verweildauer der Hefesuspension im Extraktionsteil der Anlage (Gesamtheit aller Ultraschallreaktoren) ca. 2 Stunden beträgt. Der Extraktionslösungsstrom mit der Flussrate V₂' bzw. V₃' beträgt das zwei- bis achtfache der Flussrate V₁' des Hefesuspensionsstroms, vorzugsweise das drei- bis fünffache.

Nach Durchlauf der Ultraschallreaktoren 30, 30a, 30b, 30c und 30d wird die Hefe- bzw. Hefepartikelsuspension durch ein Abtrennmittel 70 (Separator) zur Feststoffabscheidung (Feststoffstrom mit der Flussrate V₄') geführt. Der aus dem Abtrennmittel 70 (Separator) stammende feststofffreie Extraktionslösungsstrom wird mit einer Flussrate V₃' im Gegenstrom zum Hefesuspensionsstrom geführt, wobei eine Aufkonzentrierung des Extraktionsstroms mit den zellulären Inhaltsstoffen resultiert.

Die Ultraschallreaktoren sind mit Ultraschallköpfen 40 sowie einer geeigneten Filteranordnung, z.B. einem vertikal angeordneten Filter, ausgestattet. Der Energieeintrag durch den Ultraschall pro Ultraschallreaktor beträgt 1-10 KW vorzugsweise 2-5 KW.

Die in den Extraktionsbädern installierten, senkrecht angeordeten Filter 180 dienen der Abtrennung der Hefepartikel von der Extraktionslösung und besitzen eine mittlere Porenweite von 400-600 nm.

Der 30-40%-ige Feststoffstrom mit der Flussrate V₄' wird in einen Zwischenspeichertank (nicht abgebildet) gefördert und nach der Entleerung des Speichertanks 1 in diesen zurück gepumpt, um mit dem nachfolgenden Verfahrensschritt zu starten.

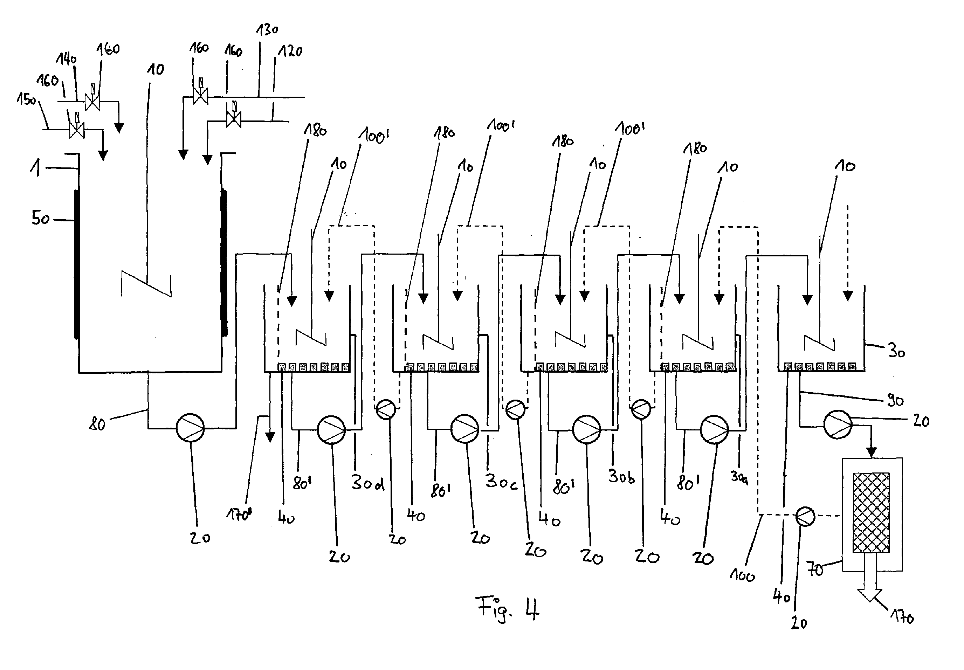

Figur 4 zeigt die kontinuierlich arbeitende Vorrichtung zur Durchführung des erfindungsgemässen Verfahrens von Figur 3.

Der Speichertank 1 mit dem Rührwerk 10 weist Zufuhrleitungen 120, 130 140 und 150 auf, die Ventile 160 umfassen. Der Speichertank ist über eine Verbindungsleitung 80 mit dem Ultraschallreaktor 30d verbunden. Die in Serie angeordneten Ultraschallreaktoren 30, 30a, 30b, 30c und 30d sind über Verbindungsleitungen 80' verbunden. Zudem weisen die einzelnen Ultraschallreaktoren je ein Rührwerk 10 sowie ultraschall-erzeugende Mittel 40 auf. Als ultraschall-erzeugende Mittel können z.B. Schallköpfe verwendet werden.

Vom Ultraschallreaktor 30 führt eine Abfuhrleitung 90 zum Abtrennmittel 70, welches einen Ausgang 170 für den Feststoffstrom und eine Rückführleitung 100 aufweist, die dazu dient, den (feststoff-freien) Extraktionslösungsstrom in den Ultraschallreaktor 30a zurückzuführen. Die Ultraschallreaktoren 30a, 30b, 30c und 30d sind über weitere Rückführungsleitungen 100' verbunden. Dabei verhindert ein in diesen Ultraschallreaktoren (30a, 30b, 30c und 30d) senkrecht angeordneter Filter 180, dass der Hefesuspensionstrom (Hefezell- bzw. Hefepartikelsuspension) in unerwünschter Weise zurück in die vorangehend angeordneten Ultraschallreaktoren gelangen kann. Der Ultraschallreaktor 30d weist zusätzlich einen Ausgang 170' auf, über welchen der Extraktionslösungsstrom weggeführt werden kann. Ausserdem weisen die Verbindungsleitungen 80, 80', die Abfuhrleitung 90 und die Rückführungsleitungen 100 und 100' je eine Pumpe 20 zur Beförderung des Hefesuspensions- bzw. des Extraktionsstroms auf.

Möglich sind auch Variationen der vorangehend beschriebenen Ausführungsformen der Vorrichtung. Zum Beispiel kann die Vorrichtung mehr als einen Speichertank aufweisen. Zudem kann auch die Zahl der Ultraschallreaktoren variiert werden.

### Beispiel 1 - Absatzweise betriebene Glucanisolierung

2 Liter einer Bierhefesuspension werden zentrifugiert, um die Hefe als ca. 40-%ige stichfeste Masse zu isolieren. Die 30-%ige Hefesuspension wird durch dreimaliges Waschen und Zentrifugieren von Salzen und anderen löslichen Bestandteilen weitgehend gereinigt.

Im Anschluss an den Reinigungsprozess wird das Hefekonzentrat in einen auf 100°C beheizten Rührkessel gegeben und während 8 Minuten Sattdampf eingeleitet. Nach abgeschlossener Dampfbehandlung wird Wasser zudosiert, um eine ca. 15-%ige Suspension herzustellen. Nach weiteren 10 Minuten Verweilzeit im gerührten Kessel wird die Suspension zentrifugiert, mit Wasser versetzt bis wieder eine ca. 15-%ige Hefesuspension vorliegt.

Im Verfahrensschritt a) folgt nun die C-Lysat-Gewinnung. Die im Rührkessel vorliegende 15-%ige Hefesuspension wird auf 45°C aufgeheizt und zirkuliert durch einen bzw. mehrere Ultraschallreaktoren zurück in den Rührkessel. Der pH-Wert wird auf 4.8 eingestellt. Der Energieeintrag durch den Ultraschall beträgt 25 W/l. Nach einstündiger Extraktionsdauer wird die Suspension zentrifugiert, um das C-Lysat von der Hefe abzutrennen. Diese Extraktionsstufe wird viermal in derselben Weise wiederholt, wobei die einzelnen C-Lysat-Extrakte vereinigt werden. Nach abgeschlossener C-Lysat-Extraktion resultiert ein Gewichtsverlust der Hefe von 56 %.

Das durch Zentrifugation isolierte Hefezellwandmaterial wird erneut mit Wasser versetzt, um eine 15-%ige Suspension herzustellen. Im Rahmen des Verfahrensschrittes b) werden eine Reaktionstemperatur von 55°C und ein pH-Wert von 8.5 durch kontinuierliche, pH-geregelte Zudosierung einer 5-%igen Natronlaugelösung eingestellt. Nach einstündiger Behandlungsdauer wird der proteinhaltige Extrakt durch Zentrifugation abgetrennt. Diese Prozessstufe wird dreimal durchgeführt, wobei im dritten Zyklus die Temperatur auf 70°C angehoben wird. Während des gesamten dritten Verfahrensschrittes wird die Suspension vom gerührten Kessel durch die Ultraschallreaktoren zirkuliert. Die hierbei eingetragene Ultraschallenergie beträgt 25 W/1. Im Anschluss an die drei Prozesszyklen des dritten Verfahrensschrittes werden die vorwiegend Protein enthaltenden Extrakte vereinigt. Der hierbei resultierende Gewichtsverlust des Hefezellwandmaterials beträgt 36% bezogen auf die nach der C-Lysat-Extraktion resultierenden Trockenmasse.

Mit dem nach dem Verfahrensschritt b) durch Zentrifugation isolierten Hefezellwandmaterial wird durch Wasserzusatz erneut eine 15-%ige Suspension hergestellt, um im Verfahrensschritt c) die Mannan- und Lipidextraktion durchzuführen. Die 15-%ige Suspension wird im Rührkessel auf 80°C aufgeheizt und kontinuierlich durch die Ultraschalleinheit zirkuliert. Der hierbei eingestellte pH-Wert beträgt 11.0-11.3. Unter den erwähnten Bedingungen werden vier Extraktionszyklen durchgeführt, die jeweils 45 Minuten dauern. Der fünfte Extraktionszyklus wird bei 80°C und bei einem pH-Wert von 9 unter Zusatz von 5 g/l Butoxyethanol durchgeführt, um noch sorbierte Restlipide zu extrahieren. Die ersten vier Extrakte, die nahezu das gesamte extrahierbare Mannan und Lipid enthalten, werden vereinigt, während der aus dem fünften Zyklus stammende Extrakt dem Abwasser zugeführt wird.

Das nun vorliegende, durch Zentrifugation isolierte, gereinigte Glucan wird in drei weiteren Zyklen gewaschen, neutralisiert und nochmals gewaschen und anschliessend im Rahmen eines Vakuum-Trocknungsprozesses entwässert. Der isolierte Glucananteil beträgt 12% bezogen auf die eingesetzte Hefemasse. Das so gewonnene Glucan ist ein weisses bis gelbliches, geruchloses Pulver, dessen Proteingehalt < 2% und Lipidgehalt <3% ist.

## Patentansprüche

1. Verfahren zur Isolierung von Glucan aus Hefe umfassend die folgenden Schritte
a) Behandlung einer Hefesuspension mit Ultraschall bei einer ersten Temperatur T1 und einem ersten pH-Wert pH1 und abtrennen eines ersten Feststoffs A;
b) Resuspendieren des ersten Feststoffs A, Behandlung des resuspendierten Feststoffs A mit Ultraschall bei einer zweiten Temperatur T2 und einem zweiten pH-Wert pH2 und abtrennen eines zweiten Feststoffs B; und
c) Resuspendieren des Feststoffs B, Behandlung des resuspendierten Feststoffs B mit Ultraschall bei einer dritten Temperatur T3 und einem dritten pH-Wert pH3 und abtrennen eines dritten Feststoffs C,
wobei T2 höher als T1 und T3 höher T2 ist.

2. Verfahren nach Anspruch 1, wobei die Hefe in einem ersten Schritt aus einem flüssigen Kulturmedium abgetrennt wird.

3. Verfahren nach einem der vorangehenden Ansprüche, wobei die Hefe gewaschen wird.

4. Verfahren nach einem der vorangehenden Ansprüche, wobei die Hefe mit Dampf behandelt wird.

5. Verfahren nach einem der vorangehenden Ansprüche, wobei der Feststoff C in wenigstens einem Zyklus gewaschen wird.

6. Verfahren nach einem der vorangehenden Ansprüche, wobei der Feststoff C in wenigstens drei Zyklen gewaschen wird und in einem der Zyklen, vorzugsweise dem zweiten, ein annähernd neutraler pH-Wert eingestellt wird.

7. Verfahren nach einem der vorangehenden Ansprüche, wobei die erste Temperatur T1 25-60°C, vorzugsweise 40-50°C, und der erste pH-Wert 3-8, vorzugsweise 4-6, beträgt.

8. Verfahren nach einem der vorangehenden Ansprüche, wobei die zweite Temperatur T2 30-80°C, vorzugsweise 50-60°C, und der zweite pH-Wert 7-12, vorzugsweise 8-9, beträgt.

9. Verfahren nach einem der vorangehenden Ansprüche, wobei die dritte Temperatur T3 70-100°C, vorzugsweise 80-90°C, und der dritte pH-Wert 10-12, vorzugsweise 10.5-11.5, beträgt.

10. Verfahren nach einem der vorangehenden Ansprüche, wobei der zweite pH-Wert pH2 höher ist als der erste pH-Wert pH1 und der dritte pH-Wert pH3 höher ist als der zweite pH-Wert pH2.

11. Verfahren nach einem der vorangehenden Ansprüche, wobei die Hefe Brau- oder Bäckerhefe ist.

12. Verfahren nach einem der vorangehenden Ansprüche, wobei das Verfahren unter Schutzgas, vorzugsweise Stickstoff, durchgeführt wird.

13. Partikuläres Glucan hergestellt nach dem Verfahren nach einem der Ansprüche 1-12, wobei das Glucan im Schritt c) als Feststoff C abtrennbar ist.

14. C-Lysat hergestellt nach dem Verfahren nach einem der Ansprüche 1-12, wobei das Lysat im Schritt a) vom Feststoff A abtrennbar ist.

15. Protein hergestellt nach dem Verfahren nach einem der Ansprüche 1-12, wobei das Protein im Schritt b) vom Feststoff B abtrennbar ist.

16. Mannan und Lipid hergestellt nach dem Verfahren nach einem der Ansprüche 1-12, wobei das Mannan und Lipid im Schritt c) vom Feststoff C abtrennbar ist.

17. Verwendung des Glucans hergestellt nach dem Verfahren nach einem der Ansprüche 1-12, als Microcontainer.

18. Vorrichtung zur Durchführung des Verfahrens nach einem der Ansprüche 1-12, umfassend einen Speichertank (1) mit einem Rührwerk (10), eine Pumpe (20) zur Beförderung einer Suspension, einen Ultraschallreaktor (30) mit einem ultraschall-erzeugenden Mittel (40), ein Mittel zur Einstellung der Temperatur (50), ein Mittel zur Einstellung des pH-Werts (60), ein Abtrennmittel (70) zur Abtrennung eines Feststoffs, eine Verbindungsleitung (80) zwischen dem Speichertank (1) und dem Ultraschallreaktor (30) und eine Abfuhrleitung (90) zwischen dem Ultraschallreaktor (30) und dem Abtrennmittel (70).

19. Vorrichtung nach Anspruch 18, wobei die Vorrichtung mindestens zwei, vorzugsweise vier, in Serie angeordnete und über Verbindungsleitungen (80) verbundene Ultraschallreaktoren (30) aufweist.
